# EUROPEAN PATENT APPLICATION

(11) **EP 4 748 937 A1**
(43) Date of publication of application: **27.05.2026**
(21) Application number: 24880099.7
(22) Date of filing: 16.10.2024
(51) Int. Cl.: C12N 15/77, C12N 9/12, C12P 13/22

(54) **MICROORGANISM INTO WHICH FOREIGN RIBOSE-PHOSPHATE DIPHOSPHOKINASE HAS BEEN INTRODUCED, AND L-TRYPTOPHAN PRODUCTION METHOD USING SAME**

(30) Priority: 17.10.2023 KR 20230138960
(71) Applicant: CJ Cheiljedang Corporation, Seoul 04560 (KR)
(72) Inventor: PARK, Soe-hee, Seoul 04560 (KR); KIM, Hye Mi, Seoul 04560 (KR); KIM, Hyun Ah, Seoul 04560 (KR); SEO, Chang Il, Seoul 04560 (KR); JUNG, Moo Young, Seoul 04560 (KR); KIM, Seon Hye, Seoul 04560 (KR)
(74) Representative: Meissner Bolte Partnerschaft mbB
(86) International application number: PCT/KR2024/015647
(87) International publication number: WO 2025/084762

(57) **Abstract**

The present disclosure relates to a *Corynebacterium* sp. microorganism having L-tryptophan-producing ability, into which ribose-phosphate pyrophosphokinase derived from *Bacillus subtilis* or a polynucleotide encoding the same has been introduced; a method for producing L-tryptophan, comprising culturing the microorganism in a medium; a composition for producing L-tryptophan, comprising the microorganism, a culture of the microorganism, a fermentation product of the microorganism, or a combination of two or more thereof; and use of the microorganism for producing L-tryptophan.

## Description

### [Technical Field]

The present disclosure relates to a *Corynebacterium* sp. microorganism having L-tryptophan-producing ability, into which ribose-phosphate pyrophosphokinase derived from *Bacillus subtilis* or a polynucleotide encoding the same has been introduced; a method for producing L-tryptophan, comprising culturing the microorganism in a medium; a composition for producing L-tryptophan, comprising the microorganism, a culture of the microorganism, a fermentation product of the microorganism, or a combination of two or more thereof; and use of the microorganism for producing L-tryptophan.

### [Background Art]

Processes for producing a desired substance (*e.g.,* an amino acid) from microorganisms are environmentally friendly and safe production methods and have been the subject of various studies. Among these, research on producing a desired substance in large quantities from *Corynebacterium* sp. microorganisms has been continuously conducted. *Corynebacterium* sp. microorganisms, particularly *Corynebacterium glutamicum,* are Gram-positive microorganisms widely used for the production of L-amino acids and other useful substances.

L-amino acids are the fundamental building blocks of proteins and are used as important materials for pharmaceutical raw materials, food additives, animal feed, nutritional supplements, insecticides, and fungicides. In order to produce L-amino acids and other useful substances, various studies have been conducted on developing high-efficiency production microorganisms and fermentation process technologies. For example, substance-specific approaches such as increasing the expression of genes encoding enzymes involved in the biosynthesis of L-tryptophan or eliminating genes unnecessary for the biosynthesis have mainly been employed (US 8945907 B2).

L-tryptophan is an essential amino acid and has been widely used as a raw material for pharmaceuticals and as a material for health foods, such as feed additives and infusion solutions. While it can be produced by chemical synthesis, enzymatic methods, fermentation methods, and the like, direct fermentation using microorganisms is currently the predominant production method. According to prior studies, it has been demonstrated through intracellular quantitative analysis that, among the 20 amino acids, tryptophan biosynthesis requires the highest level of energy (Proc. Natl. Acad. Sci. USA, (2002) Vol. 99, pp. 3695-3700). Therefore, there remains a need for research on effectively increasing the L-tryptophan-producing ability.

### [Disclosure]

### [Technical Problem]

The technical problem to be solved by the present disclosure is to provide a microorganism into which foreign ribose-phosphate pyrophosphokinase is introduced and a method for producing L-tryptophan using the same.

### [Technical Solution]

One aspect of the present disclosure provides a *Corynebacterium* sp. microorganism having L-tryptophan-producing ability, into which ribose-phosphate pyrophosphokinase derived from *Bacillus subtilis* or a polynucleotide encoding the same has been introduced.

In one specific embodiment, the ribose-phosphate pyrophosphokinase may comprise the amino acid sequence of SEQ ID NO: 1 or an amino acid sequence having at least 90% sequence identity thereto.

In another specific embodiment, the ribose-phosphate pyrophosphokinase derived from *Bacillus subtilis* may be encoded by a *prsA* gene.

In another specific embodiment, the polynucleotide encoding the ribose-phosphate pyrophosphokinase derived from *Bacillus subtilis* may comprise the base sequence of SEQ ID NO: 2.

In another specific embodiment, the *Corynebacterium* sp. microorganism may be *Corynebacterium glutamicum.*

In the microorganism according to any one of the foregoing specific embodiments, the *Corynebacterium* sp. microorganism may have increased L-tryptophan-producing ability compared to a non-modified microorganism.

Another aspect of the present disclosure provides a method for producing L-tryptophan, comprising culturing, in a medium, a *Corynebacterium* sp. microorganism having L-tryptophan-producing ability, into which ribose-phosphate pyrophosphokinase derived from *Bacillus subtilis* or a polynucleotide encoding the same has been introduced.

In one specific embodiment, the method may further comprise recovering L-tryptophan from the cultured microorganism, a culture of the microorganism, a fermentation product of the microorganism, or the culture medium.

Still another aspect of the present disclosure provides a composition for producing L-tryptophan, comprising: a *Corynebacterium* sp. microorganism having L-tryptophan-producing ability, into which ribose-phosphate pyrophosphokinase derived from *Bacillus subtilis* or a polynucleotide encoding the same has been introduced; a culture of the microorganism; a fermentation product of the microorganism; or a combination of two or more thereof.

### [Advantageous Effects]

The *Corynebacterium* sp. microorganism having L-tryptophan-producing ability of the present disclosure, into which ribose-phosphate pyrophosphokinase derived from *Bacillus subtilis* or a polynucleotide encoding the same has been introduced, can produce L-tryptophan in high yield, and therefore can be usefully employed in industrial production of L-tryptophan.

### [Detailed Description of Preferred Embodiments]

The present disclosure will be described in detail as follows. Meanwhile, each description and embodiment described herein can be applied to other descriptions and embodiments, respectively. That is, all combinations of various elements described herein fall within the scope of the present disclosure. Further, the scope of the present disclosure is not limited by the specific description described below. Additionally, a number of papers and patent documents have been cited throughout the present specification. The content of the cited papers and patent documents is incorporated herein by reference in its entirety to describe the level of the technical field to which the present disclosure belongs and the contents of the present disclosure more clearly.

### Definitions

As used in the specification and appended claims of the present disclosure, the singular articles ("a", "an", and "the") may comprise plural referents unless the context clearly indicates otherwise. Further, unless the context indicates otherwise, singular terms may comprise their plural forms, and plural terms may comprise their singular forms. Additionally, as used in the specification and appended claims of the present disclosure, the use of "or" may comprise the meaning of "and/or", unless indicated otherwise.

As used herein, the term "about" may precede a specific numerical value. As used herein, the term "about" encompasses not only the precise numerical value that is specified after the term but also a range that is approximately or nearly close to that value. Considering the context in which the number is presented, it can be determined whether the specific number mentioned is close to or nearly that number. In one example, the term "about" can refer to a range of -10% to +10% of the numerical value. As another example, the term "about" may refer to a range of -5% to +5% of the given numerical value. However, it is not limited thereto.

As used herein, terms such as "first, second, third,...", "i), ii), iii),...", or "(a), (b), (c), (d),..." may be used to distinguish each component. When these terms are used in relation to steps of a method, use, or analysis, these terms do not indicate that the steps are to be performed consecutively or in a particular order and the steps may be performed, for example, with no temporal interval between the steps, simultaneously, or sequentially, in reverse order, or in a random order with intervals of seconds, minutes, hours, days, or months.

As used herein, the term "consisting of" means that the ratio of the specific features, steps, components, or other element(s) following the term amounts to 100%. The features, steps, components, or other elements following the term "consisting of" may be essential or mandatory. For example, apart from the features, steps, components, or other elements following the term "consisting of", other arbitrary features, steps, components, or other elements, or non-essential features, steps, components, or other elements may be excluded.

As used herein, the term "consisting essentially of" may mean that, where the features, steps, components, or other elements claimed in the present disclosure are substantially unaffected by the presence of at least one unspecified feature, step, component, or another element, the at least one unspecified feature, step, component, or other element may be present.

As used herein, the term "comprising" refers to the presence of features, steps, components, or other elements following the term and does not exclude the presence of at least one additional feature, step, or component. As used herein, the specific features, steps, components, or other elements following the term "comprising" may be essential or mandatory; however, in some specific embodiments, other arbitrary or non-essential features, steps, components, or other elements may also be comprised.

### Proteins, Polypeptides

As used herein, the term "protein" or "polypeptide" refers to a polymer or oligomer of consecutive amino acid residues. In the present disclosure, "polypeptide", "protein", and "peptide" may be used interchangeably.

As used herein, the term "mature polypeptide" or "mature protein" refers to a polypeptide or protein in a form without a signal sequence or propeptide sequence. A mature polypeptide or mature protein may be in a functional form of polypeptide or protein. Mature polypeptide or mature protein refers to the final form of polypeptide after translation and/or post-translational modification. For example, examples of post-translational modification comprise N-terminal processing, C-terminal truncation, glycosylation, phosphorylation, leader sequence removal, *etc.,* but are not limited thereto.

In the present disclosure, unless indicated otherwise, amino acid sequences are described in the N-terminal to C-terminal direction.

In the present disclosure, with respect to amino acid sequences, it is apparent that a polypeptide or protein "comprising" an amino acid sequence set forth in a specific sequence number, a polypeptide or protein "consisting of" an amino acid sequence set forth in a specific sequence number, or a polypeptide or protein "having" an amino acid sequence set forth in a specific sequence number may also comprise a polypeptide or protein in which certain amino acid(s) are deleted, modified, substituted, or added, as long as it has the same or corresponding activity as the polypeptide or protein consisting of the amino acid sequence of the sequence number. For example, the polypeptide or protein may also comprise, in the internal region or the region upstream or downstream (N-terminus or C-terminus) of the polypeptide or protein, a polypeptide or protein having addition or deletion of amino acid(s), naturally-occurring mutations, silent mutations, or conservative substitutions that do not alter the functions of the protein of the present disclosure.

Further, the scope of the polypeptide or protein of an amino acid sequence set forth in a specific sequence number may also comprise, for example, a polypeptide or protein conjugated to an N-terminal signal (or leader) sequence involved in the co-translational or post-translational translocation of the protein (polypeptide) or a polypeptide or protein conjugated with another sequence or linker so as to enable determination, purification, or synthesis of the polypeptide or protein.

As used herein, the term "conservative substitution" refers to the substitution of an amino acid with another amino acid having similar structural and/or chemical properties. Such an amino acid substitution may generally occur based on similarity in polarity, charge, solubility, hydrophobicity, hydrophilicity, and/or amphipathic nature of the residues. For example, positively charged (basic) amino acids comprise arginine, lysine, and histidine; negatively charged (acidic) amino acids comprise glutamate and aspartate; amino acids with nonpolar side chains (nonpolar amino acids) comprise glycine, alanine, valine, leucine, isoleucine, methionine, phenylalanine, tryptophan, and proline; and amino acids with polar or hydrophilic side chains (polar amino acids) comprise serine, threonine, cysteine, tyrosine, asparagine, and glutamine. In another example, amino acids with electrically charged side chains (electrically charged amino acids) may comprise arginine, lysine, histidine, glutamate, and aspartate, and amino acids with electrically uncharged side chains (also referred to as uncharged amino acids or neutral amino acids) may comprise glycine, alanine, valine, leucine, isoleucine, methionine, phenylalanine, tryptophan, proline, serine, threonine, cysteine, tyrosine, asparagine, and glutamine. In another example, phenylalanine, tryptophan, and tyrosine may be categorized into aromatic amino acids. In another example, valine, leucine, and isoleucine may be categorized into branched-chain amino acids (branched amino acids). In another example, 20 types of amino acids may be classified into five groups by size, starting from the group of amino acids with relatively small volumes: glycine, alanine, and serine; cysteine, proline, threonine, aspartate, and asparagine; valine, histidine, glutamate, and glutamine; isoleucine, leucine, methionine, lysine, and arginine; and phenylalanine, tryptophan, and tyrosine. However, the classification of amino acids is not limited thereto. Typically, conservative substitution may have little or no effect on the activity of a polypeptide or protein.

### Genes, Polynucleotides

As used herein, the term "gene" refers, in a narrow sense, to a polynucleotide encoding a functional molecule, and, in a broad sense, to a polynucleotide comprising the polynucleotide encoding the functional molecule and a polynucleotide comprising a region upstream or downstream of the polynucleotide. In one specific embodiment, the functional molecule may be an RNA or a protein, and a gene may have a sequence (intron) inserted between each coding region (exon).

As used herein, the term "polynucleotide", "nucleic acid", or "nucleic acid molecule" refers to a polymer of nucleotides in which nucleotide monomers are connected in a long chain by covalent bonds, and means a DNA (*e.g.,* cDNA or genomic DNA) or RNA (*e.g.,* mRNA) strand having at least a certain length. In the present disclosure, "polynucleotide", "nucleic acid", and "nucleic acid molecule" may be used interchangeably.

### Identity, Homology

As used herein, the term "identity" or "homology" refers to the degree of similarity between two given amino acid sequences or base sequences and may be expressed as a percentage. In the present disclosure, "homology" and "identity" may often be used interchangeably.

The sequence homology or identity of conserved polynucleotides or polypeptides may be determined by a standard alignment algorithm, and default gap penalties established by the program used may be applied together.

Whether any two polynucleotide or polypeptide sequences have homology, similarity, or identity may be determined by, for example, a known computer algorithm such as the "FASTA" program (Pearson et al., (1988) [Proc. Natl. Acad. Sci. USA 85]: 2444) using default parameters. Alternatively, it may be determined by comparing the sequence information using the Needleman-Wunsch algorithm (Needleman and Wunsch, 1970, J. Mol. Biol. 48:443-453) as performed in the Needleman program of the EMBOSS package (EMBOSS: The European Molecular Biology Open Software Suite, Rice et al., 2000, Trends Genet. 16:276-277) (version 5.0.0 or later), or a GAP computer program such as the Smith-Waterman algorithm (Smith and Waterman, Adv. Appl. Math (1981) 2:482) (the GCG program package (Devereux, J. et al., Nucleic Acids Research 12:387 (1984)), BLASTP, BLASTN, and FASTA (Atschul, S. F. et al., J MOLEC BIOL 215:403 (1990); Guide to Huge Computers, Martin J. Bishop, ed., Academic Press, San Diego, 1994, and CARILLO et al. (1988) SIAM J Applied Math 48:1073) are included). For example, the homology, similarity, or identity may be determined by using BLAST of the National Center for Biotechnology Information or ClustalW.

Further, whether any two polynucleotide sequences have homology, similarity, or identity may be confirmed through Southern hybridization experiments under appropriate hybridization conditions, and the appropriate hybridization conditions can be determined by methods well known to those skilled in the art (such as J. Sambrook et al., Molecular Cloning, A Laboratory Manual; F.M. Ausubel et al., Current Protocols in Molecular Biology, John Wiley & Sons, Inc., New York), but are not limited thereto. For example, homologous or identical polynucleotide sequences can typically hybridize under stringent conditions along the whole sequence or at least about 50%, 60%, 70%, 80%, or 90% of the full length.

As used herein, the term "stringent conditions" refers to conditions that enable specific hybridization between polynucleotides. These conditions are specifically described in the literature (see Sambrook *et al.,* supra, 9.50-9.51, 11.7-11.8). For example, the stringent conditions may comprise conditions under which polynucleotides having high homology or identity, *i.e.,* polynucleotides having at least 60%, at least 70%, at least 75%, at least 80%, at least 85%, at least 90%, at least 95%, at least 96%, at least 97%, at least 98%, or at least 99% homology or identity hybridize with each other, while polynucleotides having homology or identity lower than the above homology or identity do not hybridize with each other; or may comprise ordinary washing conditions of Southern hybridization, *i.e.,* washing once, specifically twice or three times, at a salt concentration and temperature corresponding to 60°C, 1XSSC, 0.1% SDS, specifically 60°C, 0.1XSSC, 0.1% SDS, and more specifically 68°C, 0.1XSSC, 0.1% SDS.

The hybridization may occur between nucleotides having complementary nucleotide sequences, but the hybridized polynucleotides may comprise some base mismatches depending on the stringency of the hybridization. The term "complementary" is used to describe a relationship between nucleotide bases that can hybridize with each other. For example, regarding DNA, adenosine is complementary to thymine, and cytosine is complementary to guanine. Therefore, the polynucleotide of the present disclosure may comprise an isolated nucleic acid fragment complementary to the entire sequence as well as a base sequence substantially similar thereto.

For example, polynucleotides having homology or identity to the polynucleotide of the present disclosure may be detected through hybridization at a Tₘ value of 55°C. Further, the Tₘ value may be 60°C, 63°C, or 65°C, but is not limited thereto, and may be appropriately adjusted by those skilled in the art.

The appropriate stringency for hybridizing the polynucleotides depends on the length and degree of complementarity of the polynucleotides, and these variables are well known in the art (*e.g.,* Sambrook *et al.,* supra).

### Nucleic Acid Constructs, Vectors, Transformation

As used herein, the term "nucleic acid construct" refers to an artificially designed single-stranded or double-stranded nucleic acid molecule comprised in a vector that can be used to integrate a desired genetic material into an appropriate host or host cell. In one example, the nucleic acid construct may comprise a transgene delivered through a transformation vector that enables the inserted sequence to be replicated and/or expressed in a host cell. In one example, the transgene may be replicated from an existing sequence or may be artificially synthesized.

As used herein, the term "vector" refers to a DNA construct for delivering a desired polynucleotide into a suitable host or host cell.

In one example, the vector may comprise a base sequence of a polynucleotide encoding a desired polypeptide that is operably linked to a suitable expression regulatory region (or expression regulatory sequence) so as to enable the expression of the desired polypeptide in a suitable host. The expression regulatory region may comprise a promoter capable of initiating transcription, any operator sequence for regulating the transcription, a sequence encoding a suitable mRNA ribosome-binding site, and a sequence for regulating termination of transcription and translation. The vector, after being transformed into a suitable host cell (microorganism), may replicate or function independently of the host genome, or may be integrated into the genome itself to replicate or function.

Additionally, in one example, the vector of the present disclosure may comprise a sequence for inserting a desired polynucleotide into a chromosome. The insertion of a polynucleotide into a chromosome using a vector may be performed by any method known in the art, for example, homologous recombination, but is not limited thereto.

The vector used in the present disclosure is not particularly limited, and any vector known in the art may be used. Examples of commonly used vectors comprise plasmids, cosmids, viruses, and bacteriophages, either in their natural state or in a recombinant state. For example, as a phage vector or cosmid vector, pWE15, M13, MBL3, MBL4, IXII, ASHII, APII, t10, t11, Charon4A, Charon21A, *etc.,* may be used; and as a plasmid vector, those based on pDZ, pDC, pBR, pUC, pBluescriptII, pGEM, pTZ, pCL, pET, *etc.,* may be used. In one example, pDZ, pDC, pDCM2, pACYC177, pACYC184, pCL, pECCG117, pUC19, pBR322, pMW118, pCC1BAC vectors, *etc.,* may be used.

The vector may further comprise a selection marker to confirm transformation into a host cell, or further, insertion into a chromosome of the host cell. The selection marker is used to select cells transformed with the vector or to confirm the insertion of a desired polynucleotide into a chromosome; markers that confer selectable phenotypes, such as drug resistance, nutrient requirements, resistance to cytotoxic agents, or expression of surface polypeptides, may be used. In an environment treated with a selective agent, only the cells that express the selection marker survive or exhibit a different phenotype, allowing for the selection of transformed cells.

As used herein, the term "transformation" refers to the introduction of a desired polynucleotide or a vector comprising the same into a host cell (microorganism), thereby altering the genetic traits of the host cell (microorganism). The transformed polynucleotide may be inserted into the chromosome of a host cell (microorganism) or located outside the chromosome. Further, the polynucleotide may comprise DNA or RNA. The polynucleotide may be introduced in a suitable form depending on the purpose of introduction. For example, a polynucleotide for expressing a desired polypeptide may be introduced into a host cell (microorganism) in the form of an expression cassette, which is a gene construct comprising all elements required for self-expression. The expression cassette may typically comprise a promoter operably linked to the coding sequence of a desired polynucleotide, a transcription termination signal, a ribosome binding site, and a translation termination signal. The expression cassette may be in the form of an expression vector capable of self-replication. Further, the polynucleotide may be introduced into a host cell (microorganism) as-is and may be operably linked to a sequence required for expression in the host cell (microorganism), but is not limited thereto.

As used herein, the term "operably linked" refers to a configuration in which a regulatory sequence is positioned at an appropriate position to regulate the expression of a coding sequence. Thus, the term "operably linked" comprises attaching or linking a regulatory region of a functional domain having a known or desired activity, such as a promoter, a stop codon, a signal sequence, or an enhancer region, to a target (gene or polypeptide) so as to regulate the expression, secretion, or function of the target in accordance with the known or desired activity. For example, it may mean that a polynucleotide sequence encoding a polypeptide is functionally linked to a promoter sequence that initiates and mediates transcription of the polynucleotide.

As used herein, the term "expression" comprises any step involved in the production of a polypeptide, for example, transcription, post-transcriptional modification, translation, post-translational modification, secretion, and the like, but is not limited thereto.

As used herein, the term "expression vector" refers to a linear or circular nucleic acid molecule comprising a desired polynucleotide sequence and an operably linked regulatory sequence for expression thereof. For example, it may comprise a base sequence of a polynucleotide encoding a desired polypeptide that is operably linked to a suitable expression regulatory region (or expression regulatory sequence) such that the desired polypeptide can be expressed in a suitable host.

As used herein, the term "regulatory sequence" refers to a polynucleotide sequence necessary for regulating the expression of a desired polynucleotide sequence. Each regulatory sequence may be a regulatory sequence native (derived from the same origin) or foreign (derived from a different gene) to the coding sequence, a variant sequence thereof, or another artificial sequence. Examples of the regulatory sequence may comprise a leader sequence, a polyadenylation sequence, a propeptide sequence, a promoter, a signal peptide sequence, an operator sequence, a sequence encoding a ribosome-binding domain, and a sequence regulating the termination of transcription and translation. The minimum unit of the regulatory sequence may comprise a promoter and a sequence for terminating transcription and translation.

As used herein, the term "genetic recombination" refers to a natural or artificial process in which an element constituting a gene, such as DNA or RNA, is rearranged into an order different from the original sequence during disassembly and reassembly processes.

As used herein, the term "recombinant gene" refers to a gene having a new genomic configuration that results from genetic recombination, such as chemical synthesis or genetic engineering techniques. In the present disclosure, the terms "recombinant gene", "recombinant DNA", and "recombinant polynucleotide" may be used interchangeably. In one example, the recombinant gene may comprise an artificial combination of nucleic acid fragments such as regulatory sequences that are not naturally found together.

As used herein, the term "recombinant protein" refers to a protein produced as a result of genetic recombination.

### Microorganisms

As used herein, the term "microorganism (or strain)" comprises both wild-type microorganisms and prokaryotic or eukaryotic microorganisms that have undergone genetic modification naturally or artificially. It may be a microorganism in which a specific mechanism is weakened or enhanced due to insertion of a foreign gene or enhancement or inactivation of activity of an endogenous gene, and may be a microorganism comprising a genetic modification for production of a desired polypeptide, protein, or product. In the present disclosure, the terms "microorganism", "strain", "host", and "host cell" may be used interchangeably.

As used herein, the term "recombinant microorganism" refers to a microorganism that has been genetically modified to exhibit a different genotype and/or phenotype compared to a naturally occurring microorganism (*e.g.,* when genetic modification affects how the nucleic acid sequence is encoded in the microorganism), and may comprise all progeny or potential progeny of the microorganism. In the present disclosure, the terms "recombinant microorganism", "genetically modified microorganism", "recombinant host cell", "recombinant cell", and "recombinant strain" may be used interchangeably. The recombinant microorganism may, for example, express a gene that is not found in its natural (non-recombinant) form, not express a gene that is expressed in its natural form, or express a natural gene in a manner different from that in which it is expressed in its natural form.

For example, the microorganism of the present disclosure may be a microorganism (such as a recombinant microorganism) into which ribose-phosphate pyrophosphokinase or a polynucleotide encoding the same is introduced, but is not limited thereto.

As used herein, the term "microorganism having L-tryptophan-producing ability" refers to a microorganism capable of producing L-tryptophan within a living organism, and may comprise any microorganism that inherently lacked L-tryptophan-producing ability but has been imparted the ability to produce L-tryptophan, as well as any microorganism that inherently possesses L-tryptophan-producing ability. The L-tryptophan-producing ability may be imparted or enhanced by strain improvement.

As used herein, the term "non-modified microorganism (strain)" does not exclude a microorganism (strain) comprising mutations that may occur naturally, and may refer to a wild-type microorganism (strain) or a natural microorganism (strain) as-is, or a microorganism (strain) before a trait is altered by genetic mutation due to natural or artificial factors. In the present disclosure, the term "non-modified microorganism (strain)" may be used interchangeably with "microorganism (strain) before modification", "non-mutated microorganism (strain)", "parent microorganism", "parent strain", "wild-type microorganism (strain)", "reference microorganism (strain)", or "standard microorganism (strain)". The non-modified microorganism of the present disclosure may refer to a microorganism (strain) into which ribose-phosphate pyrophosphokinase or a polynucleotide encoding the same is not introduced, or a microorganism (strain) before the introduction, but is not limited thereto. Additionally, in the present disclosure, the non-modified microorganism may be a microorganism that does not comprise a polypeptide consisting of the amino acid sequence of SEQ ID NO: 1 or a polynucleotide consisting of the sequence of SEQ ID NO: 2, but is not limited thereto.

### Increased Activity of Protein (Polypeptide)

As used herein, an "increase" in protein (polypeptide) activity means that the activity of a protein (polypeptide) in a host cell (microorganism) is increased compared to its intrinsic activity. The increase may be used interchangeably with terms such as "activation", "up-regulation", "overexpression", or "enhancement". The host cell (microorganism) may be a prokaryotic or eukaryotic microorganism.

The increase in protein (polypeptide) activity may comprise both the case in which a host cell (microorganism) exhibits a protein (polypeptide) activity that it did not inherently possess, and the case in which a host cell exhibits an improved protein (polypeptide) activity compared to its intrinsic activity or its activity before modification.

For example, the case in which it "exhibits a protein (polypeptide) activity that it did not inherently possess" or the case in which it exhibits an improved protein (polypeptide) activity may result from "introduction of a protein (polypeptide)", but is not limited thereto.

As used herein, the term "introduction" of a protein (polypeptide) refers to a case in which a gene that a microorganism did not originally possess is expressed within the microorganism so that the activity of a specific protein is exhibited, or to a case in which the polypeptide activity is enhanced, increased, or improved compared to its intrinsic activity or its activity prior to modification. For example, it may result from the introduction of a gene encoding the protein (polypeptide) into a host cell (microorganism). For example, a polynucleotide encoding a specific protein (polypeptide) may be introduced into a chromosome of a host cell (microorganism), or a vector comprising the polynucleotide encoding a specific protein (polypeptide) may be introduced into a host cell (microorganism), thereby exhibiting or enhancing the activity thereof.

The "intrinsic activity" refers to the activity of a specific protein (polypeptide) originally possessed by a host cell (microorganism) before transformation or a non-modified host cell (microorganism), when the trait is altered by genetic modification due to natural or artificial factors. This term may be used interchangeably with "activity before modification".

An increase in activity of a protein (polypeptide) compared to its endogenous activity means that the activity and/or concentration (expression level) of the protein (polypeptide) in a host cell (microorganism) is enhanced compared to the activity and/or concentration (expression level) of the protein (polypeptide) originally possessed by the host cell (microorganism) before modification or a non-modified host cell (microorganism).

In one example, the increase may refer to the emergence of activity of the corresponding protein (polypeptide) that was absent, or the increase in activity or concentration of the protein, relative to the activity or concentration in the host cell (microorganism) before transformation or in a non-modified host cell (microorganism), typically by at least about 1%, at least about 10%, at least about 25%, at least about 50%, at least about 75%, at least about 100%, at least about 150%, at least about 200%, at least about 300%, at least about 400%, or at least about 500%, up to at least about 1000% or at least about 2000% or more, but is not limited thereto.

The increase in activity of a protein (polypeptide) may be achieved by introducing a foreign protein (polypeptide) or by increasing the activity of an endogenous protein (polypeptide). Whether the activity of a protein (polypeptide) has increased may be confirmed from the increase in the activity level of the protein (polypeptide), its expression level, or the amount of product resulting from the activity of the corresponding protein (polypeptide).

The increase in the activity of a protein (polypeptide) can be achieved by various methods well known in the art, and is not limited as long as the activity of the desired protein (polypeptide) can be increased compared to that of the host cell (microorganism) before modification. Specifically, it may be achieved by using genetic engineering and/or protein engineering well known to those skilled in the art, which are routine molecular biology techniques, but is not limited thereto (*e.g.,* Sitnicka et al. Functional Analysis of Genes. Advances in Cell Biology. 2010, Vol. 2. 1-16, Sambrook et al. Molecular Cloning 2012, *etc*.).

Specifically, the increase in the activity of a protein (polypeptide) of the present disclosure may result from:
1) increase in the intracellular copy number of a polynucleotide encoding the protein (polypeptide);
2) modification of a gene expression regulatory region on a chromosome encoding the protein (polypeptide) (*e.g.,* introduction of a modification on the expression regulatory region, replacement with a sequence exhibiting stronger activity, or insertion of a sequence exhibiting stronger activity);
3) modification of a base sequence encoding a start codon or the 5'-UTR of a gene transcript encoding the protein (polypeptide);
4) modification of the amino acid sequence of the protein (polypeptide) to increase the activity of the protein (polypeptide);
5) modification of a polynucleotide sequence encoding the protein (polypeptide) to increase the activity of the protein (polypeptide) (*e.g.,* modification of a polynucleotide sequence of a gene encoding the protein (polypeptide), such that the gene encodes a protein (polypeptide) modified to have increased activity);
6) introduction of a foreign protein (polypeptide) exhibiting the activity of the protein (polypeptide), or a foreign polynucleotide encoding the same;
7) codon optimization of a polynucleotide encoding the protein (polypeptide);
8) selection and modification or chemical modification of exposed regions of the protein (polypeptide) through analysis of the tertiary structure thereof;
9) regulation of the cellular localization of the protein (polypeptide); or
10) a combination of two or more selected from 1) to 9) above, but is not particularly limited thereto.

For example,
The 1) increase in the intracellular copy number of a polynucleotide encoding the protein (polypeptide) may be achieved by introducing a vector comprising the polynucleotide encoding the protein (polypeptide) operably linked to a suitable regulatory sequence into a host cell (microorganism). Alternatively, it may be achieved by introducing one copy, or two or more copies, of a polynucleotide encoding the protein (polypeptide) operably linked to a suitable regulatory sequence into a chromosome of a host cell (microorganism). The introduction into a chromosome may be performed by introducing a vector capable of inserting the polynucleotide into a chromosome of a host cell (microorganism) into the host cell (microorganism), but is not limited thereto. The vector is as described above. With respect to the polynucleotide sequence encoding the protein, the regulatory sequence may be a native sequence (of the same origin) or a foreign sequence (derived from a different gene), a variant thereof, or another artificial sequence, and may induce the expression of the polynucleotide in a host cell (microorganism).

The 2) replacement of the expression regulatory region (or expression regulatory sequence) of a gene encoding the protein (polypeptide) on the chromosome with a sequence having strong activity may be achieved, for example, by introducing a modification into the sequence through deletion, insertion, substitution, or a combination thereof to further increase the activity of the expression regulatory region, or by replacing the sequence with a sequence having stronger activity. The expression regulatory region may comprise, but is not particularly limited to, a promoter, an operator sequence, a sequence encoding a ribosome-binding site, a sequence regulating the termination of transcription and translation, and the like. In one example, it may specifically comprise replacing the original promoter with a strong promoter, but is not limited thereto.

Examples of known strong promoters comprise cj1 to cj7 promoters (US 7662943 B2), lac promoter, trp promoter, trc promoter, tac promoter, lambda phage PR promoter, PL promoter, tet promoter, gapA promoter, SPL7 promoter, SPL13 (sm3) promoter (US 10584338 B2), O2 promoter (US 10273491 B2), tkt promoter, yccA promoter, and the like, but the strong promoter is not limited thereto.

The 3) modification of a base sequence encoding a start codon or the 5'-UTR of a gene encoding the protein (polypeptide) may be achieved, for example, by modifying the base sequence such that it encodes a different initiation codon with a higher expression rate of the protein (polypeptide) compared to the endogenous initiation codon, or by modifying the base sequence such that it encodes a ribosome binding site (RBS) sequence with a higher expression rate of the protein (polypeptide) compared to the endogenous RBS sequence, but is not limited thereto.

The 4) and 5) modification of the amino acid sequence or polynucleotide sequence of the protein (polypeptide) may be achieved by introducing a modification on the sequence of the amino acid sequence of the protein (polypeptide) or the polynucleotide sequence encoding the protein (polypeptide) through deletion, insertion, substitution, or a combination thereof to enhance the activity of the protein (polypeptide), or by replacing the sequence with an amino acid sequence or polynucleotide sequence modified to have increased activity, but are not limited thereto. The replacement may specifically be performed by inserting the polynucleotide into a chromosome by homologous recombination, but is not limited thereto.

The 6) introduction of a foreign polynucleotide exhibiting the activity of the protein (polypeptide) may be achieved by introducing into a host cell (microorganism) a foreign polynucleotide encoding a protein (polypeptide) that exhibits the same/similar activity to that of the protein (polypeptide). The foreign polynucleotide is not limited by its origin or sequence, as long as it exhibits the same/similar activity to that of the protein (polypeptide). The introduction may be performed by a transformation method known in the art appropriately selected by those skilled in the art, and the expression of the introduced polynucleotide in the host cell enables the production of the protein (polypeptide), thereby increasing its activity.

The 7) codon optimization of a polynucleotide encoding the protein (polypeptide) may be achieved by codon-optimizing an endogenous polynucleotide to increase the transcription or translation within a host cell (microorganism), or by optimizing the codons of a foreign polynucleotide such that optimized transcription and translation thereof may be achieved within the host cell.

The 8) selection and modification or chemical modification of exposed regions of the protein (polypeptide) through analysis of the tertiary structure thereof may be achieved, for example, by comparing the sequence information of the protein (polypeptide) to be analyzed with a database storing the sequence information of known proteins to identify template protein candidates according to the degree of sequence similarity and confirming the structure based on the information, thereby selecting the exposed site to be modified or chemically modified and transforming or modifying the same.

The 9) regulation of the cellular localization of the protein (polypeptide) may be achieved by targeting the protein (polypeptide) to a particular intracellular organelle or particular intracellular space. For example, it may be achieved by targeting the protein (polypeptide) to the periplasm or cytoplasm through the addition or removal of a leader sequence that functions in the targeting of proteins (polypeptides), but is not limited thereto.

Such enhancement of the protein (polypeptide) activity may refer to an increase in the activity or concentration of the corresponding protein (polypeptide) relative to the activity or concentration of the protein (polypeptide) expressed in a wild-type strain or a host cell (microorganism) before modification, or an increase in the amount of product produced by the protein (polypeptide), but is not limited thereto.

The modification of a part or the entirety of a polynucleotide in the host cell (microorganism) of the present disclosure may be induced by: (a) a method using homologous recombination using a vector for chromosomal insertion or genome editing using an engineered nuclease (*e.g.,* CRISPR-Cas9), and/or (b) treatment with light, such as ultraviolet light and radiation, and/or chemicals, but is not limited thereto.

### Culturing

As used herein, the term "culturing" refers to growing a microorganism under suitably controlled environmental conditions. The culturing process may be performed in a suitable medium known in the art under suitable culturing conditions known in the art. Such a culturing process may be easily adjusted and used by those skilled in the art according to the selected microorganism. Specifically, the culturing may be batch culturing, continuous culturing, and/or fed-batch culturing, but is not limited thereto.

As used herein, the term "medium" refers to a mixed substance containing nutrients required for culturing a microorganism as a main component, and the medium supplies nutrients, growth factors, *etc.,* including water, which are indispensable for survival and development. Specifically, any medium and culture conditions may be used for culturing the microorganism of the present disclosure without particular limitation, as long as the medium is used for the common culture of microorganisms. For example, the microorganism of the present disclosure may be cultured in a common medium containing appropriate carbon sources, nitrogen sources, phosphorus sources, inorganic compounds, amino acids, and/or vitamins, *etc*., while controlling the temperature, pH, *etc.* under aerobic conditions. For example, a culture medium for a *Corynebacterium* sp. microorganism can be found in the document ["Manual of Methods for General Bacteriology" by the American Society for Bacteriology (Washington D.C., USA, 1981)].

In the present disclosure, the carbon sources comprise carbohydrates such as glucose, saccharose, lactose, fructose, sucrose, and maltose; sugar alcohols such as mannitol and sorbitol; organic acids such as pyruvic acid, lactic acid, and citric acid; or amino acids such as glutamic acid, methionine, and lysine. Additionally, natural organic nutrients such as starch hydrolysates, molasses, blackstrap molasses, rice bran, cassava, sugarcane bagasse, and corn steep liquor may be used. Specifically, carbohydrates such as glucose and sterilized pretreated molasses (*i.e.,* molasses converted into reducing sugars) may be used, and other various carbon sources in appropriate amounts may be used without limitation. These carbon sources may be used alone or in combination of two or more thereof, but are not limited thereto.

As the nitrogen sources, inorganic nitrogen sources such as ammonia, ammonium sulfate, ammonium chloride, ammonium acetate, ammonium phosphate, ammonium carbonate, and ammonium nitrate, or organic nitrogen sources such as amino acids such as glutamic acid, methionine, and glutamine, peptone, NZ-amine, meat extracts, yeast extract, malt extract, corn steep liquor, casein hydrolysate, fish or decomposition products thereof, and skim soybean cake or decomposition products thereof may be used. These nitrogen sources may be used alone or in combination of two or more thereof, but are not limited thereto.

As the phosphorus sources, monopotassium phosphate, dipotassium phosphate, or sodium-containing salts corresponding thereto may be used. As inorganic compounds, sodium chloride, calcium chloride, iron chloride, magnesium sulfate, iron sulfate, manganese sulfate, calcium carbonate, *etc.,* may be used, and in addition, amino acids, vitamins, and/or suitable precursors may be comprised. These components or precursors may be added to the medium in a batchwise or continuous manner. However, the medium is not limited thereto.

During the culturing of the microorganism of the present disclosure, the pH of the medium may be adjusted by adding a compound such as ammonium hydroxide, potassium hydroxide, ammonia, phosphoric acid, or sulfuric acid to the medium in an appropriate manner. During the culturing, an antifoaming agent such as fatty acid polyglycol ester may be used to suppress foaming. To maintain an aerobic state of the medium, oxygen or oxygen-containing gas may be injected into the medium. To maintain an anaerobic or microaerobic state of the medium, no gas may be injected, or nitrogen, hydrogen, or carbon dioxide gas may be injected. However, the culturing conditions are not limited thereto.

In the culturing of the present disclosure, the culturing temperature may be maintained at 20°C to 45°C, specifically 25°C to 40°C, and the culturing may be performed for about 10 to 160 hours, but the culturing conditions are not limited thereto.

As used herein, the term "culture" refers to a culture solution, a concentrated culture solution, a dried product of the culture solution, a culture filtrate, a concentrated culture filtrate, or a dried product of the culture filtrate obtained by culturing a specific microorganism in a culture medium. The culture solution refers to a solution comprising a specific microorganism, whereas the culture filtrate refers to a solution that substantially does not contain the specific microorganism (wherein "substantially" means that the specific microorganism separated by filtration or the like is excluded, but does not mean that the microorganism is completely absent from the filtrate). The culture is not limited in its form, and, in one example, may be in the form of a liquid, an emulsion, or a solid.

As used herein, the term "fermentation" refers to a process in which a microorganism decomposes organic matter using enzymes possessed by the microorganism, excluding putrefactive reactions. Fermentation reactions and putrefactive reactions proceed using similar processes. However, upon decomposition, fermentation produces useful substances, whereas putrefaction produces bad odors or harmful substances.

In the present disclosure, the method for obtaining a fermentation product from the microorganism is not particularly limited and can be obtained according to a method conventionally used in the relevant or similar technical field.

As used herein, the term "fermentation product" comprises not only the fermented substance itself, but also all types of substances comprising a fermentation product produced from the microorganism, comprising a substance comprising the fermented microorganism, a culture produced from the fermented microorganism, a fermentation product of the culture, a concentrated fermentation product, a dried product of the fermentation product, a filtrate of the fermentation product, a filtrate of the concentrated fermentation product, a dried product of the filtrate of the fermentation product, an extract of the fermentation product, or a dilution of the fermentation product.

### Detailed Description of the Present Disclosure

Hereinafter, the specific embodiments of the present disclosure will be described in more detail.

One aspect of the present disclosure provides a *Corynebacterium* sp. microorganism having L-tryptophan-producing ability, into which ribose-phosphate pyrophosphokinase derived from *Bacillus subtilis* or a polynucleotide encoding the same has been introduced.

As used herein, the term "ribose-phosphate pyrophosphokinase (PRSA)" refers to an enzyme that converts ribose 5-phosphate into phosphoribosyl pyrophosphate. The ribose-phosphate pyrophosphokinase of the present disclosure may be used interchangeably with "PRSA".

Specifically, the ribose-phosphate pyrophosphokinase of the present disclosure may be a protein having ribose-phosphate pyrophosphokinase activity encoded by a *prsA* gene, but is not particularly limited thereto as long as it exhibits activity corresponding to that of ribose-phosphate pyrophosphokinase. The ribose-phosphate pyrophosphokinase encoded by a *prsA* gene is known in the art, and the amino acid and polynucleotide sequences of the ribose-phosphate pyrophosphokinase can be obtained from publicly available databases, examples of which include GenBank of NCBI, but are not limited thereto.

In one example, the ribose-phosphate pyrophosphokinase derived from *Bacillus subtilis* may comprise the amino acid sequence of SEQ ID NO: 1 or an amino acid sequence having at least 60% homology or identity thereto, but is not limited thereto as long as it retains ribose-phosphate pyrophosphokinase activity. Additionally, it is apparent that even if a protein comprises an amino acid sequence of SEQ ID NO: 1 in which part of the sequence is deleted, modified, substituted, or added, the protein may fall within the scope of the present disclosure as long as the protein exhibits efficacy corresponding to that of the ribose-phosphate pyrophosphokinase. A protein having, comprising, consisting of, or essentially consisting of an amino acid sequence having at least 60%, 70%, 80%, 85%, 90%, 95%, 96%, 97%, 98%, or 99% or more homology or identity to the amino acid sequence of SEQ ID NO: 1 that exhibits equivalent efficacy to that of the ribose-phosphate pyrophosphokinase may be comprised in the ribose-phosphate pyrophosphokinase.

Additionally, the sequence of a polynucleotide sequence encoding ribose-phosphate pyrophosphokinase derived from *Bacillus subtilis* having the amino acid sequence of SEQ ID NO: 1 or an amino acid sequence having at least 60% homology or identity thereto can be obtained, for example, based on codon information known in the art. In one example, the ribose-phosphate pyrophosphokinase may be encoded by a polynucleotide having, comprising, consisting of, or essentially consisting of the sequence of SEQ ID NO: 2 or a base sequence having at least 60%, at least 70%, at least 75%, at least 80%, at least 85%, at least 90%, at least 95%, at least 96%, at least 97%, at least 98%, or at least 99% homology or identity to the sequence of SEQ ID NO: 2, but is not limited thereto. The base sequence of SEQ ID NO: 2 can be obtained from a known database, such as GenBank of NCBI, but is not limited thereto.

In the present disclosure, the term "polynucleotide (gene) comprising the base sequence of SEQ ID NO: 2" can be used interchangeably with the term "polynucleotide (gene) having the base sequence of SEQ ID NO: 2", "polynucleotide (gene) consisting of the base sequence of SEQ ID NO: 2", or *"prsA".*

The polynucleotide of the present disclosure may undergo various modifications in the coding region within the scope that does not change the amino acid sequence of the ribose-phosphate pyrophosphokinase, taking into consideration codon degeneracy or the codons preferred in an organism in which the ribose-phosphate pyrophosphokinase of the present disclosure is to be expressed. Accordingly, it is apparent that a polynucleotide that can be translated, by codon degeneracy, into a polypeptide consisting of an amino acid sequence of the ribose-phosphate pyrophosphokinase of the present disclosure or a polypeptide having at least 60% homology or identity thereto may also be comprised in the polynucleotide of the present disclosure. For example, the polynucleotide of the present disclosure may be the sequence of SEQ ID NO: 2, or a degenerate sequence thereof.

In another example, the polynucleotide of the present disclosure may have or comprise a base sequence having at least 60%, at least 70%, at least 75%, at least 80%, at least 85%, at least 90%, at least 95%, at least 96%, at least 97%, at least 98%, or at least 99% homology or identity to the sequence of SEQ ID NO: 2, or may consist of or essentially consist of a base sequence having at least 60%, at least 70%, at least 75%, at least 80%, at least 85%, at least 90%, at least 95%, at least 96%, at least 97%, at least 98%, or at least 99% homology or identity to the sequence of SEQ ID NO: 2, but is not limited thereto.

Additionally, the polynucleotide of the present disclosure may comprise, without limitation, a probe that can be prepared from a known gene sequence, for example, any sequence that encodes the ribose-phosphate pyrophosphokinase of the present disclosure by hybridizing with a sequence complementary to all or part of the polynucleotide sequence of the present disclosure under stringent conditions.

The *Corynebacterium* sp. microorganism of the present disclosure exhibits L-tryptophan-producing ability.

For the purpose of the present disclosure, the microorganism of the present disclosure may comprise any microorganism capable of producing the desired L-tryptophan, into which ribose-phosphate pyrophosphokinase or a polynucleotide encoding the same is introduced. For example, the microorganism of the present disclosure may be a microorganism characterized by having increased L-tryptophan-producing ability resulting from introducing ribose-phosphate pyrophosphokinase or a polynucleotide encoding the same thereinto, and the microorganism may be a genetically engineered microorganism or a recombinant microorganism, but is not limited thereto. Specifically, the recombinant strain with increased L-tryptophan-producing ability may be a microorganism with increased L-tryptophan-producing ability compared to a natural wild-type microorganism, a non-modified microorganism having intrinsic activity of ribose-phosphate pyrophosphokinase, or a non-modified microorganism with no intrinsic activity of ribose-phosphate pyrophosphokinase, but is not limited thereto.

In one example, the microorganism having L-tryptophan-producing ability refers to a prokaryotic or eukaryotic microorganism capable of producing L-tryptophan within a living organism, and may comprise any microorganism that exhibits increased L-tryptophan-producing ability or has been imparted L-tryptophan-producing ability by the activity of the ribose-phosphate pyrophosphokinase of the present disclosure introduced into a microorganism that has intrinsic L-tryptophan-producing ability or no L-tryptophan-producing ability. The L-tryptophan-producing ability may be imparted or enhanced by strain improvement.

The microorganism of the present disclosure may comprise any microorganism into which ribose-phosphate pyrophosphokinase or a polynucleotide encoding the same is introduced using various known methods.

In one example, the recombinant microorganism having L-tryptophan-producing ability of the present disclosure may comprise any microorganism capable of producing L-tryptophan by being transformed through a vector introducing a foreign gene encoding ribose-phosphate pyrophosphokinase of the present disclosure, specifically ribose-phosphate pyrophosphokinase derived from *Bacillus subtilis.*

For example, the microorganism producing L-tryptophan may be a microorganism into which a polynucleotide encoding a protein comprising the amino acid sequence of SEQ ID NO: 1 or a protein comprising an amino acid sequence having at least 60%, 70%, 75%, 80%, 85%, 90%, 95%, 96%, 97%, 98%, 99%, 99.5%, 99.7%, or 99.9% or more homology or identity to the amino acid sequence of SEQ ID NO: 1 is introduced.

For example, the microorganism producing L-tryptophan may be a microorganism into which a polynucleotide capable of encoding a protein comprising an amino acid sequence having at least 80% homology with the amino acid sequence of SEQ ID NO: 1 or a polynucleotide comprising the base sequence of SEQ ID NO: 2 or a base sequence having at least 60%, at least 70%, at least 75%, at least 80%, at least 85%, at least 90%, at least 95%, at least 96%, at least 97%, at least 98%, or at least 99% homology or identity to the base sequence of SEQ ID NO: 2 is introduced.

In one example, the microorganism with increased L-tryptophan-producing ability of the present disclosure may be a microorganism with increased L-tryptophan-producing ability compared to a non-modified microorganism, but is not limited thereto. In one example, the non-modified microorganism, which is the reference strain for comparing the increase in L-tryptophan-producing ability, may be the CM05-9157 strain, but is not limited thereto.

In one example, the microorganism having increased L-tryptophan-producing ability may exhibit an increase of at least about 1%, specifically, at least about 1%, at least about 2.5%, at least about 5%, at least about 6%, at least about 7%, at least about 8%, at least about 9%, at least about 10%, at least about 15%, at least about 16%, at least about 17%, at least about 18%, at least about 19%, at least about 20%, or at least about 21% (the upper limit is not particularly limited, for example, at most about 200%, at most about 150%, at most about 100%, at most about 50%, at most about 45%, at most about 40%, at most about 35%, at most about 30%, or at most about 25%) compared to the L-tryptophan-producing ability of a parent microorganism (parent strain) before modification or a non-modified microorganism. However, the L-tryptophan-producing ability of the microorganism is not limited thereto, as long as it exhibits a positive increase compared to the producing ability of a parent microorganism (parent strain) before modification or a non-modified microorganism. In another example, the recombinant microorganism having increased L-tryptophan-producing ability may have an increase of at least about 1.1-fold, at least about 1.15-fold, at least about 1.16-fold, at least about 1.17-fold, at least about 1.18-fold, at least about 1.19-fold, at least about 1.2-fold, or at least about 1.21-fold (the upper limit is not particularly limited, for example, at most about 10-fold, at most about 5-fold, at most about 3-fold, at most about 2-fold, at most about 1.5-fold, at most about 1.4-fold, at most about 1.3-fold, or at most about 1.25-fold) compared to that of a parent microorganism (parent strain) before modification or a non-modified microorganism, but is not limited thereto.

In one example, the microorganism having L-tryptophan-producing ability of the present disclosure may be a prokaryotic cell or a eukaryotic cell, and specifically may be a prokaryotic cell. The prokaryotic cell may include, for example, an *Escherichia* sp. microorganism, *Erwinia* sp. microorganism, *Serratia* sp. microorganism, *Providencia* sp. microorganism, *Corynebacterium* sp. microorganism, *Pseudomonas* sp. microorganism, *Leptospira* sp. microorganism, *Salmonella* sp. microorganism, *Brevibacteria* sp. microorganism, *Hyphomonas* sp. microorganism, *Chromobacterium* sp. microorganism, or *Norcadia* sp. microorganism, or a microorganism belonging to fungi or yeast, but is not limited thereto. Specifically, it may be an *Escherichia* sp. microorganism, *Corynebacterium* sp. microorganism, *Leptospira* sp. microorganism, or yeast. More specifically, it may be a *Corynebacterium* sp. microorganism.

In a microorganism according to any one of the foregoing specific embodiments, the microorganism of the present disclosure may be a *Corynebacterium* sp. microorganism.

In one embodiment of the present disclosure, the microorganism of the present disclosure may be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens.* Specifically, the microorganism of the present disclosure may be a *Corynebacterium* sp. microorganism, and more specifically *Corynebacterium glutamicum,* but is not limited thereto.

Specifically, the microorganism of the present disclosure may be a *Corynebacterium* sp. microorganism, and more specifically *Corynebacterium glutamicum,* but is not limited thereto.

Meanwhile, the *Corynebacterium* sp. microorganism having L-tryptophan-producing ability of the present disclosure may comprise any natural wild-type microorganism, *Corynebacterium* sp. microorganism with increased L-tryptophan-producing ability by enhancing or weakening the expression of a gene related to the L-tryptophan production mechanism, or *Corynebacterium* sp. microorganism with increased L-tryptophan-producing ability by introducing or enhancing an exogenous gene.

Another aspect of the present disclosure provides a method for producing L-tryptophan, comprising culturing, in a medium, a *Corynebacterium* sp. microorganism having L-tryptophan-producing ability of the present disclosure, into which ribose-phosphate pyrophosphokinase derived from *Bacillus subtilis* or a polynucleotide encoding the same has been introduced.

In the method of the present disclosure, culturing of the microorganism may be performed using any culturing conditions and culturing methods known in the art. Such a culturing process may be readily adjusted and used by those skilled in the art according to the selected strain.

L-tryptophan produced by the culturing of the present disclosure may be secreted into the medium or remain in cells.

In one specific embodiment, the method for producing L-tryptophan of the present disclosure may further comprise preparing the microorganism of the present disclosure, preparing a medium for culturing the microorganism, or a combination thereof (in any order), for example, prior to culturing.

In one specific embodiment, the method may further comprise recovering a desired substance, specifically L-tryptophan, from the cultured microorganism, the culture of the microorganism, the fermentation product of the microorganism, or the culture medium. The recovering step may be further comprised after the culturing.

The recovery may be collecting the desired L-tryptophan using a suitable method known in the art according to the method for culturing a microorganism of the present disclosure, for example, batch, continuous, or fed-batch culturing. For example, the recovery may involve centrifugation, filtration, treatment with a crystallized protein precipitating agent (salting-out method), extraction, sonication, ultrafiltration, dialysis, various kinds of chromatography such as molecular sieve chromatography (gel filtration), adsorption chromatography, ion exchange chromatography, and affinity chromatography, HPLC, or a combination thereof. A desired substance, specifically L-tryptophan, can be recovered from the medium or microorganism using a suitable method known in the art.

Additionally, the method for producing L-tryptophan of the present disclosure may further comprise purification. The purification may be performed using a suitable method known in the art. In one example, when the method for producing L-tryptophan of the present disclosure comprises both the recovery and purification, the recovery and purification may be performed continuously, discontinuously, in any order, simultaneously, or as one integrated step, but are not limited thereto.

In the method of the present disclosure, ribose-phosphate pyrophosphokinase, introduction, L-tryptophan, and the like are as described in the aspects above.

Still another aspect of the present disclosure provides a composition for producing L-tryptophan, comprising a *Corynebacterium* sp. microorganism having L-tryptophan-producing ability, into which ribose phosphate pyrophosphokinase derived from *Bacillus subtilis* or a polynucleotide encoding the same has been introduced, a culture of the microorganism, a fermentation product of the microorganism, or a combination of two or more thereof.

The composition of the present disclosure may further comprise any appropriate excipient that is commonly used in compositions for producing L-tryptophan. Such excipients may be, for example, a preservative, a wetting agent, a dispersing agent, a suspending agent, a buffering agent, a stabilizing agent, an isotonic agent, *etc.,* but are not limited thereto.

In one specific embodiment, the composition of the present disclosure may comprise each component in a microbially effective amount or in an amount that can be suitably present in a composition for production.

In the composition of the present disclosure, ribose-phosphate pyrophosphokinase, introduction, L-tryptophan, and the like are as described in the aspects above.

Still another aspect of the present disclosure provides use of a *Corynebacterium* sp. microorganism having L-tryptophan-producing ability, into which ribose phosphate pyrophosphokinase derived from *Bacillus subtilis* or a polynucleotide encoding the same has been introduced, for producing L-tryptophan.

In the use of the present disclosure, ribose-phosphate pyrophosphokinase, introduction, L-tryptophan, and the like are as described in the aspects above.

### [Mode for Carrying Out the Invention]

The present disclosure will be described in detail by way of Examples. However, these Examples are given for illustrative purposes only, and the scope of the present disclosure is not intended to be limited by these Examples. Meanwhile, technical descriptions absent in the present disclosure may be sufficiently understood and readily practiced by those skilled in the art of the present disclosure or related art.

### Example 1. Identification and selection of the ribose-phosphate pyrophosphokinase (prsA) gene

To identify candidate genes considered to encode enzymes that convert ribose 5-phosphate into phosphoribosyl pyrophosphate and the organisms possessing the same, a PSI-BLAST search was performed using the amino acid sequence of prsA, a ribose-phosphate pyrophosphokinase derived from *Corynebacterium glutamicum,* as the query sequence, based on the NCBI and KEGG databases. As a result, taking into consideration the biosafety level applicable to production strains and the feasibility of securing the organisms, three organisms were selected as shown in Table 1 below.

**[Table 1]**

| Order | Strain | Protein Accession Number | Genome Accession Number | Biosafety Level |
|---|---|---|---|---|
| 1 | *Bacillus subtilis* | WP_003218353.1 | NC_000964.3 | 1 |
| 2 | *Mycobacterium smegmatis* | WP_003896824.1 | NZ_CP054795.1 | 1 |
| 3 | *Escherichia coli* | WP_001115461.1 | NC_007779.1 | 1 |

### Example 2. Preparation of microorganism producing L-tryptophan, into which foreign ribose-phosphate pyrophosphokinase is introduced

### Example 2-1. Construction of plasmid for gene insertion

For gene insertion into the *Corynebacterium* chromosome, the plasmid pDCM2 (Korean Registered Patent No. 10-2278000) was used as the parent vector, and, to enhance the activity of ribose-phosphate pyrophosphokinase, a plasmid for additionally inserting a *prsA* gene was constructed using the Pcj7 promoter (Korean Registered Patent No. 10-0620092).

Specifically, using the chromosomal DNA of wild-type *Corynebacterium glutamicum* ATCC13869 as a template, the upstream region in which homologous recombination occurs on the chromosome was amplified with the primer pair of SEQ ID NOs: 9 and 10, and the downstream region was amplified with the primer pair of SEQ ID NOs: 11 and 12, after which PCR was performed to obtain each gene fragment. The PCR was performed using Solg^{™} Pfu-X DNA polymerase (SolGent Co.), and the amplification was carried out under the following conditions: denaturation at 95°C for 4 minutes, followed by 27 cycles of denaturation at 95°C for 30 seconds, annealing at 55°C for 30 seconds, and extension at 72°C for 50 seconds, and a final extension at 72°C for 5 minutes. The primer sequences used are shown in Table 2 below.

**[Table 2]**

| SEQ ID NO | Name | Sequence (5'- 3') |
|---|---|---|
| SEQ ID NO: 9 | HR1 F | aacgacggccagtgaattcTCGTTGGCCATTACCTCAT |
| SEQ ID NO: 10 | HR1 R | ctgtttAGTACTaaaccggaagggccTTATCTGCCACACCCACAA |
| SEQ ID NO: 11 | HR2 F | cggtttAGTACTaaacaggaagagccTAAAAACGGAAGAGCCACT |
| SEQ ID NO: 12 | HR2 R | ttgcatgcctgcaggtcgacGGAAGACTATCCAAGGTGG |

The upstream and downstream fragments of the chromosomal region in which homologous recombination occurs, amplified by the PCR described above, and the chromosomal transformation vector pDCM2 (Korean Registered Patent No. 10-2278000) digested with the restriction enzymes EcoRI and Sall, were cloned using the Gibson assembly method (DG Gibson et al., Nature Methods, Vol. 6, No. 5, May 2009; NEBuilder HiFi DNA Assembly Master Mix), thereby obtaining a recombinant plasmid, which was designated pDCM2-ΔTn.

### Example 2-2. Preparation of Corynebacterium sp. microorganism into which ribose-phosphate pyrophosphokinase derived from Bacillus subtilis is introduced

First, to obtain the Pcj7 promoter, PCR was performed using p117-cj7-gfp (US 7662943 B2) as a template and the primers of SEQ ID NOs:13 and 14. The polymerase used was Solg^{™} Pfu-X DNA polymerase (SolGent Co.), and the PCR amplification was carried out under the following conditions: denaturation at 95°C for 2 minutes, followed by 27 cycles of denaturation at 95°C for 20 seconds, annealing at 55°C for 40 seconds, and extension at 72°C for 30 seconds, and a final extension at 72°C for 5 minutes. The primer sequences used are as shown in Table 3 below.

**[Table 3]**

| SEQ ID NO | Name | Sequence (5'- 3') |
|---|---|---|
| SEQ ID NO: 13 | P_Pcj7_ref-F | taaggcccttccggtttagtAGAAACATCCCAGCGCTACT |
| SEQ ID NO: 14 | P_Pcj7(B.su)_ref-R | tctccgtattgattagacatGAGTGTTTCCTTTCGTTGGG |

The gene encoding ribose-phosphate pyrophosphokinase derived from *Bacillus subtilis* selected in Example 1 has the amino acid sequence of SEQ ID NO: 1. Information on the gene encoding the ribose-phosphate pyrophosphokinase and the surrounding base sequences (NC_000964.3, SEQ ID NO: 2) was obtained from the NIH GenBank. Based on the obtained base sequences, primers were synthesized to insert the gene derived from *Bacillus subtilis* into the *Corynebacterium glutamicum* genomic DNA. The ribose-phosphate pyrophosphokinase gene derived from *Bacillus subtilis* (SEQ ID NO: 2) was synthesized using the gene synthesis service of Bionics Co., Ltd., and was amplified by PCR using the primer pair of SEQ ID NOs: 15 and 16. The polymerase used was Solg^{™} Pfu-X DNA polymerase, and the PCR amplification was carried out under the following conditions: denaturation at 95°C for 2 minutes, followed by 27 cycles of denaturation at 95°C for 20 seconds, annealing at 55°C for 40 seconds, and extension at 72°C for 1 minute, and a final extension at 72°C for 5 minutes. The primer sequences used are as shown in Table 4 below.

**[Table 4]**

| SEQ ID NO | Name | Sequence (5'- 3') |
|---|---|---|
| SEQ ID NO: 15 | G_prs(B.su)-F | cccaacgaaaggaaacactcATGTCTAATCAATACGGAGA |
| SEQ ID NO: 16 | G_prs(B.su)-R | ttaggctcttcctgtttagtTTAGCTGAACAGATAGCTGA |

Next, the Pcj7 promoter region amplified as described above, the gene fragment derived from *Bacillus subtilis,* and the chromosomal transformation vector pDCM2-ΔTn digested with the restriction enzyme Scal prepared in Example 2-1 were cloned using the Gibson assembly method (DG Gibson et al., Nature Methods, Vol. 6, No. 5, May 2009; NEBuilder HiFi DNA Assembly Master Mix), thereby obtaining a recombinant plasmid, which was designated pDCM2-ΔTn::Pcj7-prsA(B.su). The cloning was carried out by mixing the Gibson assembly reagent with each gene fragment at calculated molar ratios and incubating the mixture at 50°C for 1 hour. The constructed pDCM2-ΔTn::Pcj7-prsA(B.su) vector was transformed into the tryptophan-producing strain CM05-9157 (Korean Registered Patent No. 10-2278000) by electroporation (Appl. Microbiol. Biotechnol. (1999) 52:541-545), and, after a second crossover process, a strain was obtained in which one copy of the Pcj7_prsA(B.su) gene was inserted between the transposon genes on the chromosome. The strain was identified by PCR using the primers of SEQ ID NOs: 17 and 18, which are capable of amplifying the external regions of the upstream and downstream homologous recombination sites at the gene insertion locus, respectively, and by genome sequencing. The primer sequences used are shown in Table 5 below.

**[Table 5]**

| SEQ ID NO | Name | Sequence (5'- 3') |
|---|---|---|
| SEQ ID NO: 17 | Confirm-Pcj7-prs-F | GTTCGGCTTGGATTATGG |
| SEQ ID NO: 18 | Confirm-Pcj7-prs-R | CATCAACAACAGCCTTCA |

The strain obtained by the above method was designated CM05-9157△Tn::Pcj7_prsA(b.su).

### Example 2-3. Preparation of Corynebacterium sp. microorganism into which ribose-phosphate pyrophosphokinase derived from Mycobacterium smegmatis is introduced

First, to obtain the Pcj7 promoter, PCR was performed using p117-cj7-gfp (US 7662943 B2) as a template and the primers of SEQ ID NO: 13 in Table 3 and SEQ ID NO: 19 in Table 6. The polymerase used was Solg^{™} Pfu-X DNA polymerase (SolGent Co.), and the PCR amplification was carried out under the following conditions: denaturation at 95°C for 2 minutes, followed by 27 cycles of denaturation at 95°C for 20 seconds, annealing at 55°C for 40 seconds, and extension at 72°C for 30 seconds, and a final extension at 72°C for 5 minutes.

**[Table 6]**

| SEQ ID NO | Name | Sequence (5'- 3') |
|---|---|---|
| SEQ ID NO: 19 | P_Pcj7(m.sm)_ref-R | tcggtccagtccgtggccacGAGTGTTTCCTTTCGTTGGG |

The ribose-phosphate pyrophosphokinase derived from *Mycobacterium smegmatis* selected in Example 1 has the amino acid sequence of SEQ ID NO: 3. Information on the corresponding gene and the surrounding base sequences (NZ_CP054795.1, SEQ ID NO: 4) was obtained from the NIH GenBank. Based on the obtained base sequences, primers were synthesized to insert the gene derived from *Mycobacterium smegmatis* into the *Corynebacterium glutamicum* genomic DNA. The ribose-phosphate pyrophosphokinase gene derived from *Mycobacterium smegmatis* (SEQ ID NO: 4) was synthesized using the gene synthesis service of Bionics Co., Ltd., and was amplified by PCR in the same manner as in Example 2-2 using the primer pair of SEQ ID NOs: 20 and 21. The primer sequences used are shown in Table 7 below.

**[Table 7]**

| SEQ ID NO | Name | Sequence (5'- 3') |
|---|---|---|
| SEQ ID NO: 20 | G_prs(m.sm)-F | cccaacgaaaggaaacactcGTGGCCACGGACTGGACCGA |
| SEQ ID NO: 21 | G_prs(m.sm)-R | ttaggctcttcctgtttagtTTATGCAGACCCGTCGAACA |

Next, the Pcj7 promoter region amplified as described above, the gene fragment derived from *Mycobacterium smegmatis,* and the chromosomal transformation vector pDCM2-ΔTn digested with the restriction enzyme Scal prepared in Example 2-1 were cloned using the Gibson assembly method, thereby obtaining a recombinant plasmid, which was designated pDCM2-△Tn::Pcj7-prsA(m.sm). The cloning was carried out by mixing the Gibson assembly reagent with each gene fragment at calculated molar ratios and incubating the mixture at 50°C for 1 hour. The constructed pDCM2-△Tn::Pcj7-prsA(m.sm) vector was transformed into the tryptophan-producing strain CM05-9157 (Korean Registered Patent No. 10-2278000) by electroporation, and, after a second crossover process, a strain was obtained in which one copy of the Pcj7_prsA(m.sm) gene was inserted between the transposon genes on the chromosome. The strain was identified by PCR using the primers of SEQ ID NOs: 17 and 18 in Table 5, which are capable of amplifying the external regions of the upstream and downstream homologous recombination sites at the gene insertion locus, respectively, and by genome sequencing.

The strain obtained by the above method was designated CM05-9157△Tn::Pcj7-prsA(m.sm).

### Example 2-4. Preparation of Corynebacterium sp. microorganism into which ribose-phosphate pyrophosphokinase derived from Escherichia coli is introduced

First, to obtain the Pcj7 promoter, PCR was performed using p117-cj7-gfp (US 7662943 B2) as a template and the primers of SEQ ID NO: 13 in Table 3 and SEQ ID NO: 22 in Table 8. The polymerase used was Solg^{™} Pfu-X DNA polymerase (SolGent Co.), and the PCR amplification was carried out under the following conditions: denaturation at 95°C for 2 minutes, followed by 27 cycles of denaturation at 95°C for 20 seconds, annealing at 55°C for 40 seconds, and extension at 72°C for 30 seconds, and a final extension at 72°C for 5 minutes.

**[Table 8]**

| SEQ ID NO | Name | Sequence (5'- 3') |
|---|---|---|
| SEQ ID NO: 22 | P_Pcj7(e.co)_ref-R | aaaagcttcatatcaggcatGAGTGTTTCCTTTCGTTGGG |

The ribose-phosphate pyrophosphokinase derived from *Escherichia coli* selected in Example 1 has the amino acid sequence of SEQ ID NO: 5. Information on the corresponding gene and the surrounding base sequences (NC_007779.1, SEQ ID NO: 6) was obtained from the NIH GenBank. Based on the obtained base sequences, primers were synthesized to insert the gene derived from *Escherichia coli* into the *Corynebacterium glutamicum* genomic DNA.

The ribose-phosphate pyrophosphokinase gene derived from *Escherichia coli* (SEQ ID NO: 6) was synthesized using the gene synthesis service of Bionics Co., Ltd., and was amplified by PCR in the same manner as in Example 2-2 using the primer pair of SEQ ID NOs: 23 and 24. The primer sequences used are shown in Table 9 below.

**[Table 9]**

| SEQ ID NO | Name | Sequence (5'- 3') |
|---|---|---|
| SEQ ID NO: 23 | G_prs(e.co)-F | cccaacgaaaggaaacactcATGCCTGATATGAAGCTTTT |
| SEQ ID NO: 24 | G_prs(e.co)-R | ttaggctcttcctgtttagtTTAGTGTTCGAACATGGCAG |

Next, the Pcj7 promoter region amplified as described above, the gene fragment derived from *Escherichia coli,* and the chromosomal transformation vector pDCM2-ΔTn digested with the restriction enzyme Scal prepared in Example 2-1 were cloned using the Gibson assembly method, thereby obtaining a recombinant plasmid, which was designated pDCM2-△Tn::Pcj7-prsA(e.co). The cloning was carried out by mixing the Gibson assembly reagent with each gene fragment at calculated molar ratios and incubating the mixture at 50°C for 1 hour. The constructed pDCM2-△Tn::Pcj7-prsA(e.co) vector was transformed into the tryptophan-producing strain CM05-9157 (Korean Registered Patent No. 10-2278000) by electroporation, and, after a second crossover process, a strain was obtained in which one copy of the Pcj7_prsA(e.co) gene was inserted between the transposon genes on the chromosome. The strain was identified by PCR using the primers of SEQ ID NOs: 17 and 18 in Table 5, which are capable of amplifying the external regions of the upstream and downstream homologous recombination sites at the gene insertion locus, respectively, and by genome sequencing.

The strain obtained by the above method was designated CM05-9157△Tn::Pcj7-prsA(e.co).

### Example 2-5. Preparation of Corynebacterium sp. microorganism in which ribose-phosphate pyrophosphokinase derived from Corynebacterium glutamicum is further enhanced

First, to obtain the Pcj7 promoter, PCR was performed in the same manner as in Example 2-2, using p117-cj7-gfp (US 7662943 B2) as a template and the primers of SEQ ID NO: 13 in Table 3 and SEQ ID NO: 25 in Table 10.

**[Table 10]**

| SEQ ID NO | Name | Sequence (5'- 3') |
|---|---|---|
| SEQ ID NO: 25 | P_Pcj7(c.gl)_ref-R | tgtttccagtgagcagtcatGAGTGTTTCCTTTCGTTGGG |

To further enhance the ribose-phosphate pyrophosphokinase by inserting into the *Corynebacterium glutamicum* genomic DNA the gene (NZ_CP016335.1, SEQ ID NO: 8) encoding the ribose-phosphate pyrophosphokinase (SEQ ID NO: 7) of wild-type *Corynebacterium glutamicum* ATCC 13869, PCR was performed in the same manner as in Example 2-2 using the chromosomal DNA of the wild-type *Corynebacterium glutamicum* ATCC 13869 strain as a template and the primers of SEQ ID NOs: 26 and 27. The primer sequences used are as shown in Table 11 below.

**[Table 11]**

| SEQ ID NO | Name | Sequence (5'- 3') |
|---|---|---|
| SEQ ID NO: 26 | G_prs(c.gl)-F | cccaacgaaaggaaacactcATGACTGCTCACTGGAAACA |
| SEQ ID NO: 27 | G_prs(c.gl)-R | ttaggctcttcctgtttagtTTAGGCCTCGCCCTCGAAGA |

Next, the Pcj7 promoter region amplified as described above, the gene fragment derived from *Corynebacterium glutamicum,* and the chromosomal transformation vector pDCM2-ΔTn digested with the restriction enzyme Scal prepared in Example 2-1 were cloned using the Gibson assembly method, thereby obtaining a recombinant plasmid, which was designated pDCM2-△Tn::Pcj7-prsA(C.gl). The cloning was carried out by mixing the Gibson assembly reagent with each gene fragment at calculated molar ratios and incubating the mixture at 50°C for 1 hour. The constructed pDCM2-△Tn::Pcj7-prsA(C.gl) vector was transformed into the tryptophan-producing strain CM05-9157 (Korean Registered Patent No. 10-2278000) by electroporation, and, after a second crossover process, a strain was obtained in which one copy of the Pcj7-prsA(C.gl) gene was inserted between the transposon genes on the chromosome. The strain was identified by PCR using the primers of SEQ ID NOs: 17 and 18 in Table 5, which are capable of amplifying the external regions of the upstream and downstream homologous recombination sites at the gene insertion locus, respectively, and by genome sequencing.

The strain obtained by the above method was designated CM05-9157△Tn::Pcj7_prsA(c.gl).

### Example 3. Evaluation of L-tryptophan-producing ability of Corynebacterium sp. microorganism into which foreign ribose-phosphate pyrophosphokinase is introduced

To evaluate the L-tryptophan-producing ability of the parental strain CM05-9157, into which no foreign gene had been introduced, and of the production strains in which one copy of the ribose-phosphate pyrophosphokinase gene constructed in Examples 2-2, 2-3, 2-4, and 2-5 had been additionally enhanced-namely, CM05-9157ΔTn::Pcj7_prsA(b.su), CM05-9157ΔTn::Pcj7_prsA(m.sm), CM05-9157ΔTn::Pcj7_prsA(e.co), and CM05-9157ΔTn::Pcj7_prsA(c.gl)-the strains were cultured by the following method.

Each strain was inoculated into a 250-mL corner-baffled flask containing 25 mL of seed medium and incubated with shaking at 200 rpm at 30°C for 20 hours. Then, 1 mL of the seed culture was inoculated into a 250-mL corner-baffled flask containing 25 mL of production medium and incubated with shaking at 200 rpm at 30°C for 24 hours. After completion of the incubation, the amount of L-tryptophan produced was measured by HPLC. The compositions of the seed medium and the production medium are as follows, and the L-tryptophan concentration in the culture broth of each tested strain is shown in Table 12 below.

### [Seed Medium (pH 7.0)]

Glucose 20 g, Peptone 10 g, Yeast Extract 5 g, Urea 1.5 g, KH₂PO₄ 4 g, K₂HPO₄ 8 g, MgSO₄·7H₂O 0.5 g, Biotin 100 µg, Thiamine HCl 1000 µg, Calcium Pantothenate 2000 µg, and Nicotinamide 2000 µg (per 1 L of distilled water).

### [Production Medium (pH 7.0)]

Glucose 30 g, (NH₄)₂SO₄ 15 g, MgSO₄·7H₂O 1.2 g, KH₂PO₄ 1 g, Yeast Extract 5 g, Biotin 900 µg, Thiamine HCl 4500 µg, Calcium Pantothenate 4500 µg, and CaCO₃ 30 µg (per 1 L of distilled water).

**[Table 12]**

| | OD562 | Amount of tryptophan produced (g/L) | Tryptophan yield (*100g/g, %) |
|---|---|---|---|
| CM05-9157 | 56.5 | 1.90 | 6.45 |
| CM05-9157△Tn::Pcj7_prsA(b .su) | 52.8 | 2.32 | 7.86 |
| CM05-9157△Tn::Pcj7_prsA(c. gl) | 55.9 | 1.92 | 6.51 |
| CM05-9157△Tn::Pcj7_prsA( m.sm) | 57.1 | 1.83 | 6.22 |
| CM05-9157△Tn::Pcj7_prsA(e .co) | 54.0 | 1.97 | 6.67 |

As a result, as shown in Table 12, the amount of tryptophan produced by the CM05-9157△Tn::Pcj7_prsA(c.gl) strain, in which the ribose-phosphate pyrophosphokinase gene derived from *Corynebacterium glutamicum* was additionally enhanced, was 1.92 g/L, which was almost identical to that of the parental strain CM05-9157.

In contrast, the CM05-9157ΔTn::Pcj7_prsA(b.su) strain, into which the gene derived from *Bacillus subtilis* was introduced, produced 2.32 g/L of L-tryptophan in flask culturing, which represented an approximately 21% improvement in fermentation yield compared to the CM05-9157ΔTn::Pcj7_prsA(c.gl) strain.

The CM05-9157ΔTn::Pcj7_prsA(m.sm) and CM05-9157ΔTn::Pcj7_prsA(e.co) strains, in contrast to the control strain CM05-9157, exhibited a slight decrease in tryptophan production or only a relatively marginal increase.

These results indicate that only the introduction of the ribose-phosphate pyrophosphokinase gene derived from *Bacillus subtilis,* among the foreign ribose-phosphate pyrophosphokinase genes introduced into *Corynebacterium* sp. microorganisms, can specifically increase the L-tryptophan-producing ability.

As set forth above, those skilled in the art will be able to understand that the present disclosure may be embodied in other specific forms without departing from the technical spirit or essential characteristics thereof. In this regard, it should be understood that the foregoing examples are illustrative in all respects and are not to be construed as limiting. The scope of the present disclosure should be construed as comprising the meaning and scope of the appended claims rather than the detailed description, and all changes or variations derived from the equivalent concepts fall within the scope of the present disclosure.

### [Sequence Listing]

| SEQ ID NO | Name | Sequence |
|---|---|---|
| 1 | prsA(b.su) | MSNQYGDKNLKIFSLNSNPELAKEIADIVGVQLGKCSVTRFSDGEVQI NIEESIRGCDCYIIQSTSDPVNEHIMELLIMVDALKRASAKTINIVIPYYG YARQDRKARSREPITAKLFANLLETAGATRVIALDLHAPQIQGFFDIPID HLMGVPILGEYFEGKNLEDIVIVSPDHGGVTRARKLADRLKAPIAIIDKR RPRPNVAEVMNIVGNIEGKTAILIDDIIDTAGTITLAANALVENGAKEVYA CCTHPVLSGPAVERINNSTIKELVVTNSIKLPEEKKIERFKQLSVGPLLA EAIIRVHEQQSVSYLFS |
| 2 | *prsA(b.su)* | atgtctaatcaatacggagataagaatttaaagattttttctttgaattcgaatccagagcttgcaaaa gaaatcgcagatatagttggagttcaattagggaaatgttctgtcacaagatttagtgacggggaag tccaaattaatatcgaagaaagtattcgcggatgtgattgttacatcatccagtctacaagtgacccc gttaacgagcatattatggaactgctgattatggtagatgcgttaaaacgcgcttctgcaaaaacgat taacattgttattccttattacggttatgcgcgtcaagacagaaaagcaagatcccgtgagccaatc acagctaaacttttcgctaacctgcttgaaacagccggtgcgactcgtgtgattgcacttgacctgca tgcgccgcaaattcaaggattctttgatataccgattgaccacttaatgggtgttccgattttaggaga atattttgaaggcaaaaatcttgaagatatcgtcattgtttcaccagaccatggcggtgtgacacgtg cccgcaaactggctgaccgactaaaagcgccaattgcgattatcgataaacgccgtccgcgtcca aacgtggcggaagtcatgaatattgtaggtaacatcgaagggaagactgctatcctcatcgatgac |
| | | attattgatactgcaggtacgattacacttgctgctaatgcgctcgttgaaaacggagcgaaagaag tatatgcatgctgtacacaccctgtactatcaggccctgcggttgaacggattaataattcaacaatta aagagcttgttgtgacaaacagcatcaagcttcctgaagaaaagaaaattgaacgctttaagcag ctttcagtcggaccgcttctggccgaagcgattattcgcgttcatgagcagcaatcagtcagctatct gttcagctaa |
| 3 | prsA(m.sm) | MATDWTDNRKNLMLFAGRAHPELADQVAKELDVAVTAQTARDFANG EIFVRFDESVRGCDAFVLQSHPAPLNQWLMEQLIMIDALKRGSAKRIT AILPFYPYARQDKKHRGREPISARLVADLLKTAGADRIVSVDLHTDQIQ GFFDGPVDHMRAQKLLTGYIGEHYADEDMVVVSPDSGRVRVAEKWA DSLGGVPLAFIHKTRDPLVPNQVKSNRVVGDVKGKTCILTDDMIDTGG TIAGAVNLLREDGAKDVIIAATHGVLSDPAPQRLAECGAREVIVTNTLPI TEDKRFPQLTVLSIAPLLANTIRAVFENGSVTGLFDGSA |
| 4 | *prsA(m.sm)* | gtggccacggactggaccgacaatcgcaaaaacttgatgctgttcgcgggtcgcgcgcaccccg agctggctgaccaagttgccaaagaactcgacgtggccgtcaccgcgcaaacggcacgggattt cgccaacggtgagatcttcgtccgcttcgacgaatccgtccgtggctgtgacgcattcgtgctgcag agccatccggcgccgctgaaccagtggctcatggaacagctgatcatgatcgacgcgctcaagc gtggcagcgccaagcgcatcaccgcgatcctgccgttctatccctacgcccggcaggacaagaa gcaccgcggccgcgaacccatctcggcacgcctcgtcgcggacctgctcaagaccgcgggcgc cgaccgcatcgtgtcggtcgacctgcacaccgaccagatccagggcttcttcgacggcccggtgg accacatgcgcgcgcagaagctgctcaccggctacatcggcgagcactacgccgacgaggac atggtggtggtgtcccccgattcgggccgcgtgcgtgtcgccgagaagtgggccgactcgctggg cggtgttccgctggccttcatccacaagacgcgtgacccgctggtccccaaccaggtcaagtcga accgcgtcgtgggtgacgtcaagggcaagacctgcatcctcaccgacgacatgatcgacacgg gcggcacgatcgccggcgcggtcaacctgctgcgtgaggacggcgccaaggacgtcatcatcg ccgcgacgcacggcgtgctgtcggatcccgccccgcagcggctcgccgagtgcggcgcacgcg aggtgatcgtcaccaacacgctgcccatcaccgaggacaagcgcttcccgcagctcacggtgct ctccatcgcaccgcttctggccaacaccatccgggccgtgttcgagaacggttcggtgacaggact gttcgacgggtctgcataa |
| 5 | prsA(e.co) | MPDMKLFAGNATPELAQRIANRLYTSLGDAAVGRFSDGEVSVQINEN VRGGDIFIIQSTCAPTNDNLMELVVMVDALRRASAGRITAVIPYFGYAR QDRRVRSARVPITAKVVADFLSSVGVDRVLTVDLHAEQIQGFFDVPVD NVFGSPILLEDMLQLNLDNPIVVSPDIGGVVRARAIAKLLNDTDMAIIDK RRPRANVSQVMHIIGDVAGRDCVLVDDMIDTGGTLCKAAEALKERGA KRVFAYATHPIFSGNAANNLRNSVIDEVVVCDTIPLSDEIKSLPNVRTLT LSGMLAEAIRRISNEESISAMFEH |
| 6 | *prsA(e.co)* | atgcctgatatgaagctttttgctggtaacgccaccccggaactagcacaacgtattgccaaccgcc tgtacacttcactcggcgacgccgctgtaggtcgctttagcgatggcgaagtcagcgtacaaattaa tgaaaatgtacgcggtggtgatattttcatcatccagtccacttgtgcccctactaacgacaacctgat ggaattagtcgttatggttgatgccctgcgtcgtgcttccgcaggtcgtatcaccgctgttatcccctac tttggctatgcgcgccaggaccgtcgcgtccgttccgctcgtgtaccaatcactgcgaaagtggttgc agacttcctctccagcgtcggtgttgaccgtgtgctgacagtggatctgcacgctgaacagattcag |
| | | ggtttcttcgacgttccggttgataacgtatttggtagcccgatcctgctggaagacatgctgcagctg aatctggataacccaattgtggtttctccggacatcggcggcgttgtgcgtgcccgcgctatcgctaa gctgctgaacgataccgatatggcaatcatcgacaaacgtcgtccgcgtgcgaacgtttcacaggt gatgcatatcatcggtgacgttgcaggtcgtgactgcgtactggtcgatgatatgatcgacactggc ggtacgctgtgtaaagctgctgaagctctgaaagaacgtggtgctaaacgtgtatttgcgtacgcga ctcacccgatcttctctggcaacgcggcgaacaacctgcgtaactctgtaattgatgaagtcgttgtc tgcgataccattccgctgagcgatgaaatcaaatcactgccgaacgtgcgtactctgaccctgtca ggtatgctggccgaagcgattcgtcgtatcagcaacgaagaatcgatctctgccatgttcgaacact aa |
| 7 | prsA(c.gl) | MTAHWKQNQKNLMLFSGRAHPELAEAVAKELDVNVTPMTARDFANG EIYVRFEESVRGSDCFVLQSHTQPLNKWLMEQLLMIDALKRGSAKRIT AILPFYPYARQDKKHRGREPISARLIADLMLTAGADRIVSVDLHTDQIQ GFFDGPVDHMHAMPILTDHIKENYNLDNICVVSPDAGRVKVAEKWAN TLGDAPMAFVHKTRSTEVANQVVANRVVGDVDGKDCVLLDDMIDTG GTIAGAVGVLKKAGAKSVVIACTHGVFSDPARERLSACGAEEVITTDTL PQSTEGWSNLTVLSIAPLLARTINEIFENGSVTTLFEGEA |
| 8 | *prsA(c.gl)* | atgactgctcactggaaacaaaaccaaaagaacctcatgctgttttcgggtcgtgcgcacccaga actggcagaagctgtagctaaagagctcgacgtcaacgtcaccccaatgacggcacgcgatttc gccaacggtgagatctacgtccgcttcgaggaatcagttcgtggctccgactgcttcgtcctgcagtc ccacacccagcctctcaacaagtggctcatggaacagctgctgatgatcgacgctttgaagcgtg gttccgcaaagcgcatcaccgcgatcctgccgttctacccttacgcccgccaggacaagaagcac cgcggccgcgagccaatttctgctcgcctcatcgccgacctcatgctcaccgctggcgcggaccgt atcgtgtccgtggacttgcacaccgatcagatccagggcttcttcgacggcccagtcgatcacatgc acgccatgccgatcctcaccgatcacatcaaggaaaactacaacctggacaacatctgcgtggtc tcccctgacgcaggtcgcgtgaaggttgcagagaagtgggctaacaccttgggcgatgccccaat ggcgttcgtgcacaagacccgctccaccgaggtagcaaaccaggttgtcgccaaccgcgtcgtc ggtgacgtcgacggcaaggactgcgtgcttctcgacgacatgatcgacactggcggcaccatcg ccggcgctgtgggcgtcctgaagaaggctggcgcaaagtcagtcgtcatcgcctgcacccacggt gtgttctctgacccagcccgcgagcgcctgtctgcatgcggtgctgaagaagtcatcaccaccgac accctgccacagtccaccgagggctggagcaacctgaccgttttgtcgatcgcaccgctgctggct cgcaccatcaacgagatcttcgaaaacggctccgtcaccaccctcttcgagggcgaggcctaa |
| 9 | HR1 F | aacgacggccagtgaattcTCGTTGGCCATTACCTCAT |
| 10 | HR1 R | ctgtttAGTACTaaaccggaagggccTTATCTGCCACACCCACAA |
| 11 | HR2 F | cggtttAGTACTaaacaggaagagccTAAAAACGGAAGAGCCACT |
| 12 | HR2 R | ttgcatgcctgcaggtcgacGGAAGACTATCCAAGGTGG |
| 13 | P_Pcj7_ref-F | taaggcccttccggtttagtAGAAACATCCCAGCGCTACT |
| 14 | P_Pcj7(B.su)_ref-R | tctccgtattgattagacatGAGTGTTTCCTTTCGTTGGG |
| 15 | G_prs(B.su)-F | cccaacgaaaggaaacactcATGTCTAATCAATACGGAGA |
| 16 | G_prs(B.su)-R | ttaggctcttcctgtttagtTTAGCTGAACAGATAGCTGA |
| 17 | Confirm-Pcj7-prs-F | GTTCGGCTTGGATTATGG |
| 18 | Confirm-Pcj7-prs-R | CATCAACAACAGCCTTCA |
| 19 | P_Pcj7(m.sm)_ref-R | tcggtccagtccgtggccacGAGTGTTTCCTTTCGTTGGG |
| 20 | G_prs(m.sm)-F | cccaacgaaaggaaacactcGTGGCCACGGACTGGACCGA |
| 21 | G_prs(m.sm)-R | ttaggctcttcctgtttagtTTATGCAGACCCGTCGAACA |
| 22 | P_Pcj7(e.co)_ref-R | aaaagcttcatatcaggcatGAGTGTTTCCTTTCGTTGGG |
| 23 | G_prs(e.co)-F | cccaacgaaaggaaacactcATGCCTGATATGAAGCTTTT |
| 24 | G_prs(e.co)-R | ttaggctcttcctgtttagtTTAGTGTTCGAACATGGCAG |
| 25 | P_Pcj7(c.gl)_ref-R | tgtttccagtgagcagtcatGAGTGTTTCCTTTCGTTGGG |
| 26 | G_prs(c.gl)-F | cccaacgaaaggaaacactcATGACTGCTCACTGGAAACA |
| 27 | G_prs(c.gl)-R | ttaggctcttcctgtttagtTTAGGCCTCGCCCTCGAAGA |

## Claims

1. A *Corynebacterium* sp. microorganism having L-tryptophan-producing ability, into which ribose-phosphate pyrophosphokinase derived from *Bacillus subtilis* or a polynucleotide encoding the same has been introduced.

2. The microorganism of claim 1, wherein the ribose-phosphate pyrophosphokinase comprises an amino acid sequence of SEQ ID NO: 1 or an amino acid sequence having at least 90% sequence identity thereto.

3. The microorganism of claim 1, wherein the ribose-phosphate pyrophosphokinase derived from *Bacillus subtilis* is encoded by a prsA gene.

4. The microorganism of claim 1, wherein the polynucleotide encoding the ribose-phosphate pyrophosphokinase derived from *Bacillus subtilis* comprises a base sequence of SEQ ID NO: 2.

5. The microorganism of claim 1, wherein the *Corynebacterium* sp. microorganism is *Corynebacterium glutamicum.*

6. The microorganism of any one of claims 1 to 5, wherein the microorganism exhibits increased L-tryptophan-producing ability compared to a non-modified microorganism.

7. A method for producing L-tryptophan, comprising culturing, in a medium, a *Corynebacterium* sp. microorganism having L-tryptophan-producing ability, into which ribose-phosphate pyrophosphokinase derived from *Bacillus subtilis* or a polynucleotide encoding the same has been introduced.

8. The method of claim 7, wherein the method further comprises recovering L-tryptophan from the cultured microorganism, a culture of the microorganism, a fermentation product of the microorganism, or the culture medium.

9. A composition for producing L-tryptophan, comprising a *Corynebacterium* sp. microorganism having L-tryptophan-producing ability, into which ribose-phosphate pyrophosphokinase derived from *Bacillus subtilis* or a polynucleotide encoding the same has been introduced, a culture of the microorganism, a fermentation product of the microorganism, or a combination of two or more thereof.

10. Use of a *Corynebacterium* sp. microorganism having L-tryptophan-producing ability, into which ribose-phosphate pyrophosphokinase derived from *Bacillus subtilis* or a polynucleotide encoding the same has been introduced, for producing L-tryptophan.
